# NOUVEAU FASCICULE DE BREVET EUROPEEN

(11) **EP 0 635 273 B2**
(45) Date de publication et mention de la décision concernant l'opposition: **17.08.2005**
(45) Mention de la délivrance du brevet: 15.12.1999
(21) Numéro de dépôt: 94401640.1
(22) Date de dépôt: 18.07.1994
(51) Int. Cl.: A61L 2/18, C01B 15/037, A01N 59/00

(54) **Procédé de traitement d'un article**
Verfahren zur Behandlung von Gegenständen
Process for treating an article

(30) Priorité: 22.07.1993 FR 9309061
(43) Date de publication de la demande: 25.01.1995
(73) Titulaire: ARKEMA, 92800 Puteaux (FR)
(72) Inventeur: Grimberg, Aurélie, D-18057 Rostock (DE); Declerck, Gérard, F-95210 Saint-Gratien (DE); Rabillier, Jean-Marc, F-78280 Guyancourt (FR); Soumarmon, Raymond, F-93130 Noisy le Sec (FR)

(56) Documents cités:
- EP-A- 0 265 381
- WO-A-79/01074
- WO-A-90/01034
- FR-A- 1 271 518
- FR-A- 1 449 711
- GB-A- 2 199 245
- JP-A- 59 221 227
- JP-A- 63 110 122
- US-A- 3 122 417
- US-A- 3 234 140
- US-A- 3 387 939
- 29th session of the Joint FAO/WHO Expert Committee on Food Additives, Geneve, 3-12 June 1985, pages 103-105
- Kirk-Othmer, Encyclopedia of Chemical Technology,Third Edition, Vol. 13, pages 12-15
- Technical Bulletin # 53-48(E)ME1, Dequest Phosphonates by Monsanto, 1991, pages 1-16

## Description

La présente invention est relative à un procédé de traitement d'un article par aspersion sur l'une au moins de ses surfaces d'une solution aqueuse de peroxyde d'hydrogène, en vue notamment de désinfecter ou stériliser ledit article.

Il est connu que la désinfection ou la stérilisation d'articles tel des aliments ou des emballages comme des emballages de produits cosmétiques, de produits pharmaceutiques, ou d'aliments peuvent être réalisées au moyen de solutions aqueuses de peroxyde d'hydrogène. A cet effet, on peut, par exemple, tremper l'article à traiter dans un bain constitué par une solution aqueuse de peroxyde d'hydrogène ou asperger l'article au moyen d'une telle solution aqueuse.

Généralement, pour une meilleure efficacité, la solution aqueuse de peroxyde d'hydrogène est utilisée à chaud.

Les procédés par aspersion permettent un contact optimum de la solution aqueuse de peroxyde d'hydrogène avec toutes les surfaces de l'article traité.

Après aspersion, l'article est habituellement séché par exemple avec de l'air chaud stérile pour éliminer toute trace de peroxyde d'hydrogène de la surface traitée. L'aspersion est effectuée avec un système d'aspersion, par exemple des buses d'aspersion. Lorsque la solution aqueuse de peroxyde d'hydrogène est aspergée à chaud sur l'article, le système d'aspersion peut comprendre, à la sortie des buses d'aspersion, un dispositif du type Thermocoax, comportant un tube et un four coaxial, permettant de porter la solution aqueuse de peroxyde d'hydrogène à la température désirée. Ce tube peut comporter de manière connue en soi des moyens permettant une meilleure dispersion de la solution aqueuse de peroxyde d'hydrogène sur la surface de l'article traité. Un tel moyen peut consister notamment en des ressorts et des vrilles.

Cependant, les solutions de peroxyde d'hydrogène habituellement mises en oeuvre dans ce type de procédé entraînent la formation d'un dépôt, d'où un encrassement progressif du système d'aspersion et un grippage des pièces mécaniques mobiles.

Il est alors nécessaire de nettoyer régulièrement, généralement une fois par jour ou, au mieux, une fois tous les deux jours, le système d'aspersion, faute de quoi ce dernier se bouche complètement. Or un nettoyage aussi fréquent du système d'aspersion oblige à arrêter la chaîne de traitement ce qui entraîne naturellement une diminution de rendement, ainsi qu'une augmentation des coûts de production.

La demanderesse a pu mettre en évidence que l'encrassement du système d'aspersion pouvait être attribué, en grande partie, à la nature de l'agent de stabilisation contenu dans la solution aqueuse de peroxyde d'hydrogène.

Ces agents de stabilisation consistent classiquement en l'acide phosphorique, des oxydes d'étain, tel le stannate de sodium, l'acide dipicolinique, le pyrophosphate acide de sodium, ou des acides phosphoniques organiques ou leur sels. Les acides phosphoniques organiques ne sont habituellement utilisés qu'en association avec d'autres agents stabilisants en particulier des oxydes d'étain. De telles associations sont par exemple décrites dans le brevet FR-1.473.779. L'utilisation d'acides phosphoniques organiques non associés à d'autres agents stabilisants n'a été décrite que pour des procédés très particuliers. Ainsi le brevet US 3.234.140 décrit des bains de traitement à base de peroxyde d'hydrogène dilué pour le blanchiment de matériaux cellulosiques. Le pH de ces bains est ajusté à une valeur comprise entre 7,5 et 12,5 et ils sont stabilisés avec des acides phosphoniques organiques. Le peroxyde d'hydrogène utilisé pour une telle application est de qualité industrielle, c'est-à-dire non débarrassé de ses composés carbonés et des ses métaux provenant de son procédé de préparation.

Le document WO 90/01034 décrit l'utilisation, comme stabilisant de peroxyde d'hydrogène, un acide organique phosphorique seul.

**L'**objet de la présente invention consiste alors en un procédé de traitement d'un article par aspersion d'une solution aqueuse de peroxyde d'hydrogène permettant de réduire notablement le grippage des pièces mécaniques mobiles et l'encrassement du système d'aspersion, et donc d'éviter ainsi un nettoyage trop fréquent de ce dernier avant qu'il ne se bouche.

La présente invention consiste en un procédé de traitement d'un article par aspersion sur l'une au moins de ses surfaces d'une solution aqueuse de peroxyde d'hydrogène, caractérisé en ce que la solution aqueuse de peroxyde d'hydrogène aspergé est stabilisée exclusivement par un acide phosphonique organique dont le résidu sec, mesuré après évaporation à 110°C, est inférieur à 100 mg/kg.

Ce procédé permet d'une manière surprenante de diminuer l'encrassement du système d'aspersion et donc d'éviter à avoir à le nettoyer tous les jours, ou au mieux, une fois tous les deux jours. Il a ainsi pu être constaté qu'un nettoyage hebdomadaire du système d'aspersion pouvait être tout à fait suffisant, cela en évitant tout risque de bouchage. De plus, les acides phosphoniques organiques utilisés comme seuls agents stabilisants, permettent de conférer une très bonne stabilité à la solution aqueuse de peroxyde d'hydrogène.

Dans le cadre de la présente invention, l'acide phosphonique organique peut être choisi dans le groupe constitué par
(i) les composés de formule (I)

   N-(CR₁R₂ - PO₃H₂)₃ (I)

   dans laquelle R₁ et R₂, identiques ou différents représentent l'hydrogène ou un radical alkyle en C₁ - C₄,
   et
(ii) les composés de formule (II) : 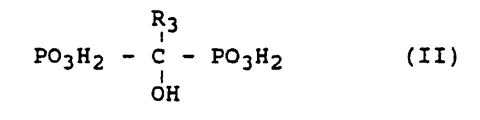
dans laquelle R₃ représente l'hydrogène ou un radical alkyle en C₁ - C₄.

A titre de composés de formule (II) ci-dessus, on peut citer, l'acide-1-hydroxyéthylène-1,1,diphosphonique.

L'acide phosphonique organique préféré pour la mise en oeuvre du procédé selon l'invention est l'acide amino-tris-méthylène-phosphonique, de formule :

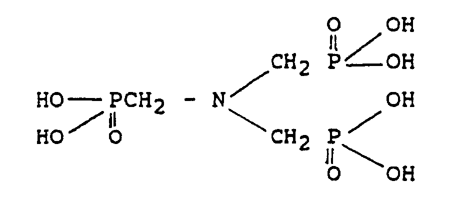

Un acide phosphonique organique tout particulièrement préféré consiste en l'acide -1- hydroxyéthylène -1, 1, diphosphonique.

Habituellement, la concentration en acide phosphonique organique dans la solution aqueuse de peroxyde d'hydrogène est, selon l'invention, inférieure à 50 mg/kg et de préférence, elle est comprise entre 10 et 30 mg/kg.

La concentration en peroxyde d'hydrogène dans ladite solution aqueuse peut être comprise entre 15% et 70%, en poids, de préférence entre 30% et 40% en poids, et plus préférentiellement cette concentration est de l'ordre de 35% en poids.

Selon l'invention, le pH apparent de la solution aqueuse de peroxyde d'hydrogène utilisé peut être inférieur à 3, et de préférence compris entre 1 et 2,7.

Selon un aspect tout particulièrement avantageux du procédé de l'invention, la solution aqueuse de peroxyde d'hydrogène stabilisée présente une grande pureté. De telles solutions aqueuses comportent avantageusement un résidu sec, mesuré après évaporation à 110°C, compris entre 20 et 35 mg/kg. Par ailleurs, la conductivité d'une telle solution de haute pureté est inférieure à 120 µS/cm, et de préférence, elle est comprise entre 60 et 100 µS/cm; tout préférentiellement, cette conductivité est de l'ordre de 80 µS/cm.

Ces solutions aqueuses de peroxyde d'hydrogène stabilisées par un acide phosphonique organique présentant une très grande pureté peuvent être obtenues à partir d'une solution aqueuse de peroxyde d'hydrogène elle-même pure, c'est-à-dire substantiellement débarrassée de ses impuretés métalliques et composés carbonés. Celle-ci peut être préparée selon des procédés bien connus de l'homme du métier, comme par exemple par distillation de solution aqueuse de peroxyde d'hydrogène de qualité industrielle.

Lorsque le procédé de traitement selon la présente invention consiste en une désinfection ou stérilisation d'un article, la solution aqueuse de peroxyde d'hydrogène est avantageusement aspergée à chaud sur ledit article. Ainsi, la température de cette solution aqueuse de peroxyde d'hydrogène à la sortie du système d'aspersion peut se faire à plus de 70°C, et de préférence à une température comprise entre 190°C et 220°C. A des températures de cet ordre, la solution aqueuse de peroxyde d'hydrogène se présente sous la forme d'un brouillard, qui entre au contact de la surface de l'article à traiter.

L'article traité selon le procédé de l'invention peut être un aliment ou un article en aluminium, en carton ou en un polymère tel le polyéthylène téréphtalate ou, de préférence, le polyéthylène. Cet article peut plus particulièrement consister en un emballage pour un aliment, un produit cosmétique ou produit pharmaceutique. Le procédé selon l'invention convient plus particulièrement à la désinfection ou la stérilisation de tels articles.

Une solution aqueuse de peroxyde d'hydrogène de haute pureté, stabilisée exclusivement par un acide phosphonique organique, caractérisée en ce que le pH apparent de ladite solution est inférieur à 3, de préférence compris entre 1 et 2,7, le résidu obtenu après évaporation à 110°C est supérieur ou égal à 20 mg/kg et inférieur à 100 mg/kg et, de préférence, inférieur ou égal à 50 mg/kg et la conductivité est comprise entre 60 µS/cm et 100 µS/cm, de préférence 80 µS/cm, convient parfaitement pour la mise en oeuvre du procédé selon l'invention.

A titre d'acide phosphonique organique pouvant être utilisé pour stabiliser la solution aqueuse de peroxyde d'hydrogène selon l'invention on peut citer les composés de formule (I) et (II) mentionnés ci-dessus.

La concentration en acide phosphonique dans ladite solution aqueuse est généralement inférieure à 50 mg/kg et de préférence elle est comprise entre 10 et 30 mg/kg. La concentration en poids en peroxyde d'hydrogène peut être comprise entre 15 et 70 % et de préférence entre 30 et 40 %, et plus préférentiellement encore, elle est de l'ordre de 35 %.

La solution aqueuse de peroxyde d'hydrogène peut être préparée à partir d'une solution aqueuse de peroxyde d'hydrogène de haute pureté à laquelle on ajoute une quantité choisie d'un acide phosphonique organique tels ceux indiqués ci-dessus. Lorsque la solution aqueuse de peroxyde d'hydrogène de haute pureté présente une concentration importante en peroxyde d'hydrogène, par exemple une concentration de 35% ou de 70 %, on peut, si nécessaire, la diluer avec une eau qui présente elle-même une grande pureté, par exemple de l'eau distillée.

Les exemples qui suivent ont pour but d'illustrer la présente invention.

### Exemple 1

En vue de la stérilisation d'emballages dont la surface est constituée d'une feuille de polyéthylène, on utilise un système d'aspersion comportant une buse d'aspersion suivie d'un Thermocoax dont le diamètre interne est de 3,5 cm et la longueur de 25 cm. Chaque emballage est aspergé avec 0,75 ml d'une solution aqueuse de peroxyde d'hydrogène distillée à 35% en poids et stabilisé par de l'acide amino-tris-méthylènephosphonique (ATMP) en une concentration de 26 mg/kg. A la sortie du Thermocoax, la solution aqueuse est à 200°C.

Le résidu sec de cette solution aqueuse stabilisée, mesurée après évaporation à 110°C, est de 20 mg/kg, sa conductivité est de 80 µS/cm et son pH apparent de 2,5.

Après plus d'une semaine d'utilisation, aucun bouchage du système d'aspersion n'a été constaté. La mise en oeuvre de la solution aqueuse de peroxyde d'hydrogène décrite ci-dessus permet de nettoyer le système d'aspersion qu'une seule fois par semaine.

### Exemple 2 (comparatif)

On reproduit l'exemple 1 avec une solution aqueuse de peroxyde d'hydrogène distillée à 35 % similaire, mais stabilisée par 9 mg/kg d'une solution aqueuse de stannate de sodium à 40 %. Le résidu sec de cette solution aqueuse, mesuré après évaporation à 110°C, est de 23 mg/kg, sa conductivité de 40 µS/cm et son pH de 3,1.

L'utilisation de cette solution aqueuse entraîne un bouchage du système d'aspersion au bout d'environ 35 heures. Dans ces conditions il est nécessaire de nettoyer le système d'aspersion une fois toutes les 24 heures.

### Exemple 3 (comparatif)

On reproduit l'exemple 1 avec une solution aqueuse de peroxyde d'hydrogène distillée similaire, mais stabilisée par 18 mg/kg de pyrophosphate acide de sodium et 2,2 mg/kg d'une solution aqueuse à 40 % de stannate de sodium. Le pH de cette solution est de 3,1.

L'utilisation de cette solution aqueuse entraîne un bouchage du système d'aspersion au bout d'environ 45 heures, ce qui oblige à le nettoyer une fois toutes les 24 heures.

### Exemple 4

On reproduit l'exemple 1 avec une solution de peroxyde d'hydrogène distillée à 35% similaire mais stabilisée par de l'acide 1-hydroxy éthylène 1-1 diphosphonique (AHEP) en une concentration de 26 mg/kg.

Le résidu sec de cette solution stabilisée, mesurée après évaporation à 110°C, est de 25mg/kg et son pH est de 2,6.

Après plus d'une semaine d'utilisation, aucun bouchage du système d'aspersion n'a été constaté. Lorsque le système d'aspersion a été nettoyé au moyen d'agents nettoyants classiques, il a été constaté que le nettoyage était plus aisé avec la solution de peroxyde d'hydrogène stabilisée par 26 mg/kg d'AHEP qu'avec 26 mg/kg d'ATMP (solution de l'exemple 1).

On entend ici par "nettoyage plus aisé", une meilleure dissolution du dépôt dans les agents nettoyants, un dépôt plus friable et une répartition plus homogène du dépôt sur les différents pièces du système d'aspersion.

### Exemple 5

En vue de déterminer la stabilité de différentes solutions aqueuses de peroxyde d'hydrogène, on a mesuré leur perte relative en titre d'H₂O₂ après 16 heures à 96°C.

La solution aqueuse de peroxyde d'hydrogène distillée à 35% avant ajout de l'agent stabilisant, utilisée dans les exemples 1 à 4, a présenté dans ces conditions une perte relative de 5,4 %.

Les solutions aqueuses stabilisées utilisées dans les exemples 1, 2, 3 et 4 ont présenté des pertes relatives de, respectivement, 1,1 %, 1 %, 3% et 1,1%.

## Revendications

1. Procédé de traitement d'un article par aspersion sur l'une au moins de ses surfaces d'une solution aqueuse de peroxyde d'hydrogène **caractérisé en ce que** ladite solution aqueuse de peroxyde d'hydrogène aspergée est stabilisée exclusivement par un acide phosphonique organique, et que son résidu sec mesuré après évaporation à 110°C est inférieur à 100 mg/kg.

2. Procédé selon la revendication 1, **caractérisé en ce que** l'acide phosphonique organique est choisi dans le groupe constitué par :
(i) les composés de formule (I)
N - (CR₁R₂ - PO₃ H₂)₃ (I)
dans laquelle R₁ et R₂, identiques ou différents représentent l'hydrogène ou un radical alkyle en C₁ - C₄, et
(ii) les composés de formule (II) 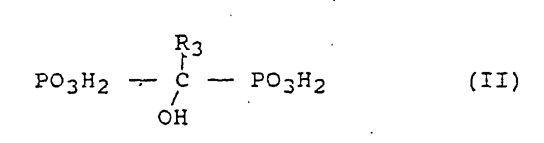
dans laquelle R₃ représente l'hydrogène ou un radical alkyle en C₁ - C₄.

3. Procédé selon l'une des revendications 1 et 2, **caractérisé en ce que** l'acide phosphonique organique est l'acide amino-tris-methylène-phosphonique.

4. Procédé selon l'une des revendications 1 et 2, **caractérisé en ce que** l'acide phosphonique organique est l'acide 1-hydroxy éthylène 1-1 diphosphonique.

5. Procédé selon l'une des revendications 1 à 4, **caractérisé en ce que** la concentration en acide phosphonique organique dans ladite solution aqueuse est inférieure à 50 mg/kg, et de préférence est comprise entre 10 et 30 mg/kg.

6. Procédé selon l'une des revendications 1 à 5, **caractérisé en ce que** la concentration en poids en peroxyde d'oxygène dans ladite solution aqueuse est comprise entre 15% et 70 %, de préférence entre 30 % et 40 %, et plus préférentiellement de l'ordre de 35 %.

7. Procédé selon l'une des revendications 1 à 6 **caractérisé en ce que** le résidu sec de ladite solution aqueuse est compris entre 20 et 50 mg/kg.

8. Procédé selon l'une des revendications 1 à 7 **caractérisé en ce que** la conductivité de la solution aqueuse est inférieure à 120 µS/cm, de préférence comprise entre 60 et 100 µS/cm, et plus préférentiellement elle est de l'ordre de 80 µS/cm.

9. Procédé selon l'une des revendications 1 à 8 **caractérisé en ce que** la dite solution aqueuse est chauffée à une température supérieure à 70°C, de préférence comprise entre 190°C et 220°C.

10. Procédé selon l'une des revendications 1 à 9 **caractérisé en ce que** le pH de ladite solution aqueuse est inférieur à 3, de préférence compris entre 1 et 2,5.

11. Procédé selon l'une des revendications 1 à 10 **caractérisé en ce que** l'article est un aliment ou un article, tel un emballage, en carton, en aluminium, ou en un matériau polymère tel le polyéthylène ou le polyéthylène-térephtalate.

## Patentansprüche

1. Verfahren zur Behandlung eines Gegenstands durch Besprühen mindestens einer seiner Oberflächen mit einer wäßrigen Wasserstoffperoxidlösung, **dadurch gekennzeichnet, daß** die versprühte wäßrige Wasserstoffperoxidlösung ausschließlich durch eine organische Phosphonsäure stabilisiert ist und einen nach Verdampfen bei 110°C bestimmten Trockenrückstand von weniger als 100 mg/kg aufweist.

2. Verfahren nach Anspruch 1, **dadurch gekennzeichnet, daß** die organische Phosphonsäure aus der Gruppe bestehend aus:
(i) Verbindungen der Formel (I)
N - (CR₁R₂ - PO₃ H₂)₃ (I)
worin R₁ und R₂ gleich oder verschieden sind und für Wasserstoff oder einen C₁-C₄-Alkylrest stehen, und
(ii) Verbindungen der Formel (II) 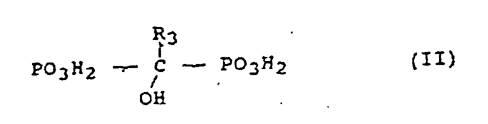
worin R₃ für Wasserstoff oder einen C₁-C₄-Alkylrest steht,
stammt.

3. Verfahren nach Anspruch 1 oder 2, **dadurch gekennzeichnet, daß** es sich bei der organischen Phosphonsäure um Aminotrismethylenphosphonsäure handelt.

4. Verfahren nach Anspruch 1 oder 2, **dadurch gekennzeichnet, daß** es sich bei der organischen Phosphonsäure um 1-Hydroxyethylen-1,1-diphosphonsäure handelt.

5. Verfahren nach einem der Ansprüche 1 bis 4, **dadurch gekennzeichnet, daß** die Konzentration an organischer Phosphonsäure in der wäßrigen Lösung unter 50 mg/kg und vorzugsweise zwischen 10 und 30 mg/kg liegt.

6. Verfahren nach einem der Ansprüche 1 bis 5, **dadurch gekennzeichnet, daß** die Wasserstoffperoxidkonzentration in der wäßrigen Lösung zwischen 15 und 70 Gew.-%, vorzugsweise zwischen 30 und 40 Gew.-% und besonders bevorzugt bei ungefähr 35 Gew.-% liegt.

7. Verfahren nach einem der Ansprüche 1 bis 6, **dadurch gekennzeichnet, daß** der Trockenrückstand der wäßrigen Lösung zwischen 20 und 50 mg/kg liegt.

8. Verfahren nach einem der Ansprüche 1 bis 7, **dadurch gekennzeichnet, daß** die Leitfähigkeit der wäßrigen Lösung unter 120 µS/cm, vorzugsweise zwischen 60 und 100 µS/cm und besonders bevorzugt bei ungefähr 80 µS/cm liegt.

9. Verfahren nach einem der Ansprüche 1 bis 8, **dadurch gekennzeichnet, daß** man die wäßrige Lösung auf eine Temperatur von mehr als 70°C und vorzugsweise zwischen 190°C und 220°C erhitzt.

10. Verfahren nach einem der Ansprüche 1 bis 9, **dadurch gekennzeichnet, daß** der pH-Wert der wäßrigen Lösung unter 3 und vorzugsweise zwischen 1 und 2,5 liegt.

11. Verfahren nach einem der Ansprüche 1 bis 10, **dadurch gekennzeichnet, daß** es sich bei dem Gegenstand um ein Lebensmittel oder einen Gegenstand, wie eine Verpackung, aus Pappe, Aluminium oder einem Polymermaterial wie Polyethylen oder Polyethylenterephthalat handelt.

## Claims

1. Process for treating an article by spraying an aqueous hydrogen peroxide solution on at least one of its surfaces, **characterized in that** the said sprayed aqueous hydrogen peroxide solution is stabilized exclusively by an organic phosphonic acid, and **in that** its dry residue, measured after evaporation at 110°C, is less than 100 mg/kg.

2. Process according to Claim 1, **characterized in that** the organic phosphonic acid is chosen from the group consisting of:
(i) compounds of formula (I)
N-(CR₁R₂ - PO₃H₂)₃ (I)
in which R₁ and R₂, which are identical or different, represent hydrogen or a C₁-C₄ alkyl radical; and
(ii) compounds of formula (II) 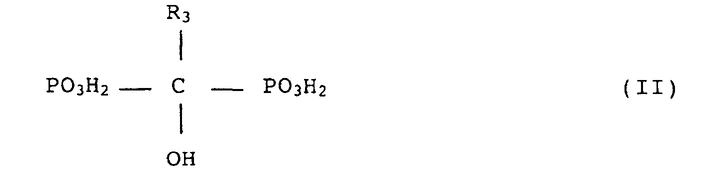
in which R₃ represents hydrogen or a C₁-C₄ alkyl radical.

3. Process according to either of Claims 1 and 2, **characterized in that** the organic phosphonic acid is amino-tris-methylene-phosphonic acid.

4. Process according to either of Claims 1 and 2, **characterized in that** the organic phosphonic acid is 1-hydroxyethylene-1,1-diphosphonic acid.

5. Process according to one of Claims 1 to 4, **characterized in that** the organic phosphonic acid concentration in the said aqueous solution is less than 50 mg/kg and preferably between 10 and 30 mg/kg.

6. Process according to one of Claims 1 to 5, **characterized in that** the hydrogen peroxide concentration by weight in the said aqueous solution is between 15% and 70%, preferably between 30% and 40% and more preferably about 35%.

7. Process according to one of Claims 1 to 6, **characterized in that** the dry residue of the said aqueous solution is between 20 and 50 mg/kg.

8. Process according to one of Claims 1 to 7, **characterized in that** the conductivity of the aqueous solution is less than 120 µS/cm, preferably between 60 and 100 µS/cm and more preferably it is about 80 µS/cm.

9. Process according to one of Claims 1 to 8, **characterized in that** the said aqueous solution is heated to a temperature greater than 70°C, preferably between 190°C and 220°C.

10. Process according to one of Claims 1 to 9, **characterized in that** the pH of the said aqueous solution is less than 3, preferably between 1 and 2.5.

11. Process according to one of Claims 1 to 10, **characterized in that** the article is a foodstuff or an article such as a package made of board, aluminium or a polymer material such as polyethylene or polyethylene terephthalate.
